# EUROPEAN PATENT APPLICATION

(11) **EP 3 933 411 A1**
(43) Date of publication of application: **05.01.2022**
(21) Application number: 20763053.4
(22) Date of filing: 19.02.2020
(51) Int. Cl.: G01N 37/00, C12M 1/00, C12M 1/34, G01N 35/08, G01N 35/10

(54) **MICROFLUIDIC DEVICE AND SAMPLE ANALYSIS METHOD**

(30) Priority: 27.02.2019 JP 2019034228
(71) Applicant: Toppan Printing Co., Ltd., Tokyo 110-0016 (JP)
(72) Inventor: GOTO, Keisuke, Tokyo 110-0016 (JP); MAKINO, Yoichi, Tokyo 110-0016 (JP)
(74) Representative: Plasseraud IP
(86) International application number: PCT/JP2020/006479
(87) International publication number: WO 2020/175264

(57) **Abstract**

There is provided a microfluidic device (1) including a flow path (35), and one or more droplet holders (11) connected to the flow path (35). In the microfluidic device (1), the flow path (35) has a height of more than 0 µm and 30 µm or less. There is also provided a sample analysis method that uses the microfluidic device (1). The method includes introducing a sample-containing aqueous solution into the flow path and allowing the droplet holders to accommodate the aqueous solution, introducing a sealant into the flow path for replacement with the aqueous solution present in the flow path and encapsulating the aqueous solution in the droplet holders, causing a reaction in the droplet holders and generating signals for detection, and detecting the signals.

## Description

### [Technical Field]

The present invention relates to microfluidic devices and sample analysis methods using microfluidic devices.

The present application claims the benefit of priority from Japanese Patent Application No. 2019-034228 filed February 27, 2019, the contents of which are incorporated herein by reference.

### [Background Art]

In recent years, studies have been performed on microwell arrays having various fine flow-path structures formed by etching techniques or photolithography techniques which are used in semiconductor manufacturing, or by methods of fine plastic molding. Wells of these microwell arrays are used as chemical reaction vessels in which various biochemical or chemical reactions are caused in fluids having small volumes.

Materials used for preparing microfluidic systems include hard materials, such as silicon and glass, and soft materials including various polymer resins, such as PDMS (polydimethylsiloxane), and silicone rubber. For example, PTLs 1 to 3 and NPL 1 describe using such microfluidic systems for various microchips and biochips.

Techniques for detecting biomolecules in flow path devices are known. For example, in DNA microarray techniques, biomolecules are introduced into micropores and heated to cause reaction to detect biomolecules. Also, techniques for detecting single biomolecules are known. For example, the techniques enabling detection of single molecules may include digital measurement techniques, such as digital ELISA (digital enzyme-linked immunosorbent assay), digital PCR (digital polymerase chain reaction), and digital ICA (digital invasive cleavage assay).

In the digital PCR technique, a mixture of reagents and nucleic acids is divided into innumerable microdroplets, and PCR amplification is performed so that signals such as fluorescence are detected from droplets containing the nucleic acids, and the number of droplets from which the signals have been detected is counted for quantification.

As methods for producing the microdroplets, a method of forming microdroplets by dividing a mixture of reagents and nucleic acids using a sealant, or a method of forming microdroplets by disposing a mixture of reagents and nucleic acids in pores formed on a substrate and then supplying a sealant are being studied.

### [Citation List]

### [Patent Literatures]

[PTL 1] JP 6183471 B
[PTL 2] JP 2014-503831 T
[PTL 3] WO2013/151135

### [Non Patent Literature]

Kim S. H., et al., Large-scale femtoliter droplet array for digital counting of single biomolecules, Lab on a Chip, 12 (23), 4986-4991, 2012.

### [Summary of the Invention]

### [Solution to Problem]

However, when optically detecting an object using fluorescence or the like by heating microdroplets formed, air bubbles may be generated in the flow path and may prevent detection.

The present invention aims to provide a microfluidic device which is capable of reducing or preventing generation of air bubbles and improving detection efficiency when optically detecting an object by heating microdroplets.

Furthermore, the present invention aims to provide a sample analysis method with which generation of air bubbles can be reduced or prevented and the efficiency of detecting a sample can be improved when optically detecting the sample by heating microdroplets.

### [Solution to Problem]

To achieve the above aims, the present invention adopts the following configurations.
[1] A microfluidic device including a flow path, and droplet holders formed in the flow path, wherein the flow path has a height of more than 0 µm and 30 µm or less.
[2] The microfluidic device according to [1], wherein the flow path includes a flat substrate, and the droplet holders are pores that are present on the substrate.
[3] The microfluidic device according to [1] or [2], wherein there are a plurality of droplet holders.
[4] The microfluidic device according to any one of [1] to [3] further including a cover member, wherein the flow path is a space sandwiched between the cover member and the substrate.
[5] The microfluidic device according to any one of [1] to [4], wherein each of the droplet holders has a volume of 10 fL or more and 100 pL or less.
[6] The microfluidic device according to any one of [1] to [5], wherein the total volume of the droplet holders is 0.2 µL or more and 2.0 µL or less.
[7] The microfluidic device according to any one of [1] to [6], wherein the ratio of the total volume of the droplet holders to the volume of the flow path is 5% or more and 40% or less.
[8] The microfluidic device according to any one of [2] to [7], wherein the ratio of the depth of the droplet holders to the height of the flow path is 3% or more and 150% or less.
[9] The microfluidic device according to any one of [2] to [8], wherein the ratio of the total area of openings of the droplet holders per unit area of the region where the droplet holders are formed in the substrate is 23% or more and 90% or less.
[10] A sample analysis method that uses the microfluidic device according to any one of [1] to [9], including: a sample supply step of supplying a sample-containing aqueous solution into the flow path and allowing the droplet holders to accommodate the aqueous solution; a sealing step of introducing a sealant into the flow path for replacement with the aqueous solution present in the flow path and encapsulating the aqueous solution in the droplet holders; a reaction step of causing a reaction in the droplet holders and generating a signal for detection; and a detection step of detecting the signal.
[11] The sample analysis method according to [10], wherein the sample is a biomolecule.
[12] The sample analysis method according to [10] or [11], wherein, in the reaction step, the microfluidic device is heated to cause the reaction, and the heating temperature is 60°C or more.
[13] The sample analysis method according to any one of [10] to [12], wherein the signal is detected using imaging.
[14] The sample analysis method according to any one of [10] to [13], wherein the signal is fluorescence.
[15] The sample analysis method according to any one of [10] to [14], wherein the reaction is an isothermal reaction.

Another aspect of the present invention encompasses the following modes.
[16] A microfluidic device including a flow path, and one or more droplet holders connected to the flow path, wherein the flow path has a height of more than 0 µm and 30 µm or less.
[17] The microfluidic device according to [16] further including a flat substrate, wherein the flow path is located above the flat substrate, and the droplet holders are present on the substrate.
[18] The microfluidic device according to [17] further including a cover member, wherein the flow path is a space sandwiched between the cover member and the substrate.
[19] The microfluidic device according to [17] or [18], wherein the ratio of the total area of openings of the droplet holders per unit area of the region where the droplet holders are formed in the substrate is 23% or more and 90% or less.
[20] The microfluidic device according to any one of [16] to [19], wherein there are a plurality of droplet holders.
[21] The microfluidic device according to any one of [16] to [20], wherein each of the droplet holders has a volume of 10 fL or more and 100 pL or less.
[22] The microfluidic device according to any one of [16] to [21], wherein the total volume of the droplet holders is 0.2 µL or more and 2.0 µL or less.
[23] The microfluidic device according to any one of [16] to [22], wherein the ratio of the total volume of the droplet holders to the volume of the flow path is 5% or more and 40% or less.
[24] The microfluidic device according to any one of [16] to [23], wherein the ratio of the depth of the droplet holders to the height of the flow path is 3% or more and 150% or less.
[25] A sample analysis method that uses the microfluidic device according to any one of [16] to [24], including: introducing a sample-containing aqueous solution into the flow path and allowing the droplet holders to hold the aqueous solution; introducing a sealant into the flow path for replacement with the aqueous solution present in the flow path and encapsulating the aqueous solution in the droplet holders; causing a reaction in the droplet holders and generating a signal for detection; and detecting the signal.
[26] The sample analysis method according to [25], wherein the sample is a biomolecule.
[27] The sample analysis method according to [25] or [26], wherein generating a signal for the detection includes causing the reaction by heating the microfluidic device, and the temperature when heating the microfluidic device is 60°C or more.
[28] The sample analysis method according to any one of [25] or [27], wherein the signal is detected by capturing an image of the microfluidic device.
[29] The sample analysis method according to any one of [25] or [28], wherein the signal is fluorescence.
[30] The sample analysis method according to any one of [25] or [29], wherein the reaction is an isothermal reaction.

### [Advantageous Effects of the Invention]

According to the microfluidic device of the present invention, generation of air bubbles can be reduced or prevented when heating microdroplets formed.

Furthermore, according to the sample analysis method of the present invention, when heating microdroplets formed to optically detect a sample, generation of air bubbles can be reduced or prevented and the efficiency of detecting the sample can be improved.

### [Brief Description of the Drawings]

Fig. 1 is a perspective view illustrating a microfluidic device according to an embodiment of the present invention.
Fig. 2 is a cross-sectional view taken along the line b-b of Fig. 1.
Fig. 3 is a cross-sectional view taken along the line b-b of Fig. 1.
Fig. 4 is a plan view illustrating a microfluidic device including a substrate in which droplet holders are formed in a region that is a part of the substrate, according to an embodiment of the present invention.
Fig. 5 is a cross-sectional view illustrating a microfluidic device according to an embodiment of the present invention.
Fig. 6 is a diagram illustrating a microfluidic device in use according to an embodiment of the present invention.
Fig. 7 is a diagram illustrating a microfluidic device in use according to an embodiment of the present invention.
Fig. 8 is a diagram illustrating a microfluidic device in use according to a comparative example.
Fig. 9A is a fluorescence image observed of a microfluidic device according to an embodiment of the present invention.
Fig. 9B is a fluorescence image observed of a microfluidic device according to a comparative example.

### [Description of the Embodiments]

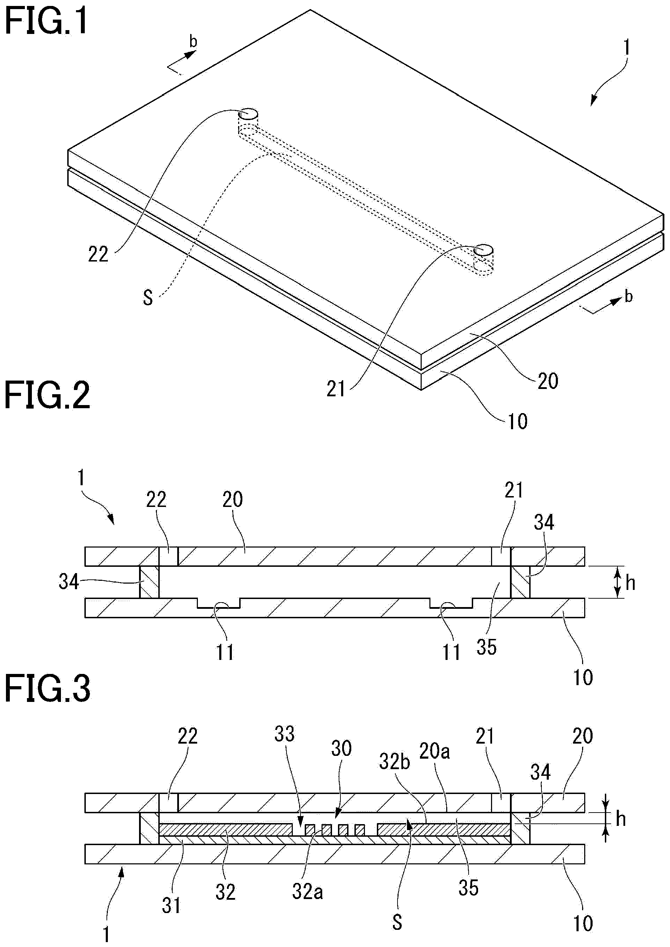

Referring to Figs. 1 to 5, an embodiment of the present invention will be described. In the following description, the dimensions in the drawings may be exaggerated for the purpose of illustration, and may not necessarily be to scale.f the drawings for ease of explanation and is not necessarily to scale.

Fig. 1 is a perspective view illustrating a microfluidic device 1 according to the present embodiment. Figs. 2 and 3 are a cross-sectional views taken along the line b-b of Fig. 1. As shown in Figs. 2 and 3, the microfluidic device 1 includes a flow path 35 and droplet holders 11. The flow path 35 may be formed by arranging a cover member 20 and a substrate 10 so as to face each other with a given interval therebetween. In this case, the flow path 35 is a space sandwiched between the cover member 20 and the substrate 10. There is a peripheral member 34 arranged between the substrate 10 and the cover member 20. The flow path 35 is a space sandwiched between the substrate 10 and the cover member 20 and enclosed by the peripheral member 34. The peripheral member 34 may be formed integrally with the cover member 20.

If the substrate 10 forming the flow path 35 is flat, i.e., a flat plate, the droplet holders 11 are preferred to be pores, i.e., wells, that are present in the substrate 10.

As shown in Fig. 3, the droplet holders 11 are preferred to be microwells 33 which are a plurality of pores (wells) formed on the substrate 10. In other words, the substrate 10 is preferred to have a plurality of microwells 33.

Hereinafter, an example of the microfluidic device 1 according to the present embodiment will be described. In the example, the microfluidic device 1 includes microwells 33, as the droplet holders 11, which are a plurality of pores (wells) on the substrate 10.

A microwell array 30 may include a bottom layer 31, a wall layer 32 (which may be termed a partition 32), and a plurality of microwells 33. The bottom layer 31 is provided on the substrate 10. The wall layer 32 is formed on the bottom layer 31. The plurality of microwells 33 are configured by the bottom layer 31 and a plurality of through holes 32a formed in the thickness direction of the bottom layer 31 and the wall layer 32. The plurality of microwells 33 are formed in an array in the wall layer 32. In an inner space S between the substrate 10 and the cover member 20, there is a gap between the microwell array 30 and the cover member 20, i.e., between the upper surface of the wall layer 32 and the cover member 20. This gap serves as the flow path 35 that allows the plurality of microwells 33 to communicate with a first hole 21 and a second hole 22.

The substrate 10 may transmit electromagnetic waves. The electromagnetic waves herein include X-rays, ultraviolet rays, visible light, infrared light, or the like. By virtue of the substrate 10 being capable of transmitting electromagnetic waves, fluorescence, phosphorescence, or the like, which is generated by reaction of the sample with the reagent encapsulated in the microfluidic device 1, can be observed from the substrate 10 side.

The substrate 10 may transmit only electromagnetic waves in a predetermined wavelength range. For example, to determine the presence of the sample in the microwells by detecting fluorescence having a peak in a wavelength range of 350 nm to 700 nm, that is a visible light region, a substrate that can transmit visible light in at least the above wavelength range may be used as the substrate 10.

Materials for forming the substrate 10 may, for example, be glass, resins, and the like. Examples of the resin substrate may include an ABS resin, polycarbonate resin, COC (cycloolefin copolymer), COP (cycloolefin polymer), acrylic resin, polyvinyl chloride, polystyrene resin, polyethylene resin, polypropylene resin, polyvinyl acetate, PET (polyethylene terephthalate), and PEN (polyethylene naphthalate). These resins may include various additives. Examples of the additives may include antioxidants, additives imparting repellency, and additives imparting hydrophilicity. The resin substrate may contain only one resin from among the above resins, or may contain a mixture of a plurality of resins.

Since fluorescence or phosphorescence may be used in a sample analysis method described later, the substrate 10 is preferred to be made of a material that does not substantially have autofluorescence. The phrase "not substantially have autofluorescence" refers to the substrate not having autofluorescence at all at wavelengths used for detecting experimental results, or having autofluorescence which is negligible and does not affect detection of the experimental results. For example, autofluorescence which is 1/2 or less or, preferably, approximately 1/10 or less the fluorescence of an object to be detected can be regarded as being insignificant and not affecting the detection of experimental results.

The substrate 10 may have a thickness that can be appropriately determined, but may be preferred to have a thickness, for example, of 5 millimeters (mm) or less, more preferably 2 mm or less, and even more preferably 1.6 mm or less. The substrate 10 may have a thickness with the lower limit being appropriately determined, but is preferred to have a thickness that does not cause strain even when the internal pressure of the microfluidic device 1 increases. For example, the thickness is preferred to be 0.1 mm or more, more preferred to be 0.2 mm or more, and even more preferred to be 0.4 mm or more. The upper limit and the lower limit of the thickness of the substrate 10 can be arbitrarily determined. The thickness of the substrate 10 is preferred, for example, to be 0.1 mm or more and 5 mm or less, more preferred to be 0.2 mm or more and 2 mm or less, and even more preferred to be 0.4 mm or more and 1.6 mm or less.

The bottom layer 31 configures the bottom surfaces of the microwells 33. Accordingly, if hydrophilicity is desired to be imparted to the bottom surfaces, the bottom layer 31 may be formed using a hydrophilic material. It is preferred that the bottom layer 31 is formed so as to permit electromagnetic waves to pass therethrough and so that the sample in the microwells 33 is not prevented from being observed from the substrate 10 side. Also, if hydrophobicity is desired to be imparted to the bottom surfaces, the bottom layer 31 may be formed using a hydrophobic material. It is preferred that the bottom layer 31 is formed so as not to prevent observation of the sample in the microwells 33 from the substrate 10 side. Furthermore, it is preferred that a material substantially having no autofluorescence is used for the bottom layer 31. Integration of the substrate 10 and the bottom layer 31 may simply be termed a substrate.

If the characteristics of the bottom surfaces of the microwells 33 can be the same as those of the substrate 10, the wall layer 32 may be directly formed on the substrate 10 without providing the bottom layer 31. Accordingly, in this case, the microwells 33 are defined by the surface of the substrate 10 and the through holes 32a of the wall layer 32.

The wall layer 32 has a plurality of through holes 32a provided in an array as viewed in the thickness direction. The inner surfaces of the respective through holes 32a constitute the inner wall surfaces of the respective microwells 33.

As the material for forming the wall layer 32, a resin or the like similar to the material forming the substrate 10 can be used, but a resin mixed with colored components that absorb electromagnetic waves of a predetermined wavelength may be used.

Considering the characteristics or the like required for the microwells 33, the resin material may be either of a hydrophilic resin in which the molecules of the resin component have a hydrophilic group and a hydrophobic resin in which the molecules of the resin component have a hydrophobic group.

Examples of the hydrophilic group may include a hydroxyl group, carboxyl group, sulfone group, sulfonyl group, amino group, amide group, ether group, and ester group. For example, the hydrophilic resin may be appropriately selected from a siloxane polymer; epoxy resin; polyethylene resin; polyester resin; polyurethane resin; polyacrylic amide resin; polyvinyl pyrrolidone resin; acrylic resin such as polyacrylic acid copolymer; polyvinyl alcohol resin such as cationized polyvinyl alcohol, silanolated polyvinyl alcohol, and sulfonated polyvinyl alcohol; polyvinyl acetal resin; polyvinyl butyral resin; polyethylene polyamide resin; polyamide polyamine resin; cellulose derivatives such as hydroxy methyl cellulose and methyl cellulose; polyalkylene oxide derivatives such as polyethylene oxide and polyethylene oxide-polypropylene oxide copolymer; maleic anhydride copolymer; ethylene-vinyl acetate copolymer; styrenebutadiene copolymer; and combinations of these resins, and the like.

For example, the hydrophobic resin, that is, a material with a contact angle of 70 degrees or more as measured according to the sessile drop method prescribed in JIS R 3257-1999, may be appropriately selected from a novolac resin; acrylic resin; methacrylic resin; styrene resin; vinyl chloride resin; vinylidene chloride resin; polyolefin resin; polyamide resin; polyimide resin; polyacetal resin; polycarbonate resin; polyphenylene sulfide resin; polysulfone resin; fluororesin; silicone resin; urea resin; melamine resin; guanamine resin; phenolic resin; cellulose resin; and combinations of these resins, and the like. Specifically, the hydrophobicity in the present specification refers to having a contact angle of 70 degrees or more as measured according to the sessile drop method prescribed in JIS R 3257-1999. The contact angle may be measured according to a method prescribed in ASTM D5725-1997 instead of the sessile drop method prescribed in JIS R 3257-1999.

The hydrophilic resin and the hydrophobic resin may both be a thermoplastic resin or a thermosetting resin. Alternatively, resins curable with ionizing radiation such as an electronic beam or UV light and elastomers may also be used.

If a photoresist is used as the resin material, numerous micro through holes 32a can be formed in the wall layer 32 with high precision using photolithography.

If photolithography is used, a photoresist to be used can be selected, applied, and exposed, and further, unwanted photoresist can be removed by appropriately selecting known methods.

If no photoresist is used, the wall layer 32 may be formed, for example, using injection molding or the like.

As the colored components, organic or inorganic pigments may be used, for example. Specifically, black pigments may be carbon black, acetylene black, iron black, and the like. Yellow pigment may be chrome yellow, zinc yellow, yellow ocher, Hansa yellow, permanent yellow, and benzine yellow. Orange pigments may be orange lake, molybdenum orange, and benzine orange. Red pigments may be red iron oxide (Bengara), cadmium red, antimony red, permanent red, resole red, lake red, brilliant scarlet, and thio-indigo red. Blue pigments may be ultramarine, cobalt blue, phthalocyanine blue, ferrocyan blue, and indigo. Green pigments may be chrome green, viridian naphthol green, phthalocyanine green, and the like.

If the wall layer 32 is formed using injection molding or the like, not only the pigment dispersed in the resin but also various dyes dissolved in the resin may be used as colored components. Dyes can be exemplified by various dye methods. Specifically, direct dyes, basic dyes, cationic dyes, acidic dyes, mordant dyes, acidic mordant dyes, sulfur dyes, vat dyes, naphthol dyes, disperse dyes, reaction dyes, and the like may be mentioned. When dying resins in particular, disperse dyes are often used.

The cover member 20 (which may also be simply termed a lid 20) is a member formed into a plate shape or a sheet shape. The cover member 20 faces the substrate 10 with an interval therebetween. In other words, the cover member 20 covers the plurality of microwells 33. The flow path 35 is a space enclosed by the cover member 20, the microwell array 30, and the peripheral member 34. The flow path 35, which communicates with the plurality of microwells 33, is located above the plurality of microwells 33.

The cover member 20 has a first hole 21 and a second hole 22 passing therethrough in the thickness direction. In plan view of the cover member 20, the first and second holes 21 and 22 are arranged sandwiching one or more droplet holders mentioned above. In a completed microfluidic device 1, the first and second holes 21 and 22 communicate with the internal space S including the microwell array 30 and the flow path 35. The first hole 21 and the second hole 22 respectively serve as an inlet through which a fluid is supplied into the internal space S and as an outlet through which the fluid is discharged.

The materials forming the cover member 20 and the thickness of the cover member 20 can be similar to those of the substrate 10.

If the cover member 20 is required to have electromagnetic wave transmissivity, electromagnetic wave transmissivity can be provided as appropriate. For example, if the step of irradiation using electromagnetic waves, which will be described later, is not performed from the cover member 20 side, the cover member 20 does not have to have electromagnetic wave transmissivity.

As shown in Fig. 2, the flow path 35 has a height h from the uppermost surface of the substrate 10 facing the flow path 35 (the surface of the substrate 10 facing the flow path 35 in a portion where no pore is formed) to the surface of the cover member 20 facing the flow path 35. As shown in Fig. 3, if the microfluidic device 1 has a wall layer 32, the height h is from a surface 32b of the wall layer 32 facing the flow path 35 to a surface 20a of the cover member 20 facing the flow path.

The height h of the flow path 35 is more than 0 µm and less than 50 µm, preferably more than 0 µm and 30 µm or less, more preferably 2 µm or more and 30 µm or less, even more preferably 3 µm or more and 25 µm or less, still more preferably 5 µm or more and 20 µm or less, and most preferably 8 µm or more and 18 µm or less. If the height of the flow path is more than 0 µm and less than 50 µm, generation of air bubbles may be minimized when heating droplets formed in the droplet holders 11 (microwells 33). Accordingly, detection of fluorescence or the like is not prevented by air bubbles and thus the sample can be detected using fluorescence or the like.

From the perspective of minimizing generation of air bubbles, for the height h of the flow path, if it is less than 50 µm, the smaller the better. However, if the height h of the flow path is less than 2 µm, the sample cannot be necessarily filled in the flow path with ease. If the height of the flow path is 2 µm or more, the sample-containing aqueous solution can be easily filled in the flow path without allowing excessively high pressure needing to be applied to the aqueous solution. If the height h of the flow path is 50 µm or more, air bubbles may be easily generated when heating the droplets formed in the droplet holders 11 (microwells 33) and the generated air bubbles may make it difficult to detect the sample using fluorescence or the like. In other words, if the height h of the flow path is less than 50 µm, air bubbles are less likely to be generated even when the droplets formed in the droplet holders 11 (microwells 33) are heated, and thus the sample can be accurately detected using fluorescence or the like. Consequently, the flow path having a height h of less than 50 µm may lead to improving productivity of the microfluidic device. In addition, if the height h of the flow path is less than 50 µm, the efficiency of detecting the sample may be improved and thus the success rate of detection may be improved.

The height of the flow path 35 may be an average of the heights at a plurality of positions on the uppermost surface of the substrate 10 facing the flow path 35 (the surface of the substrate 10 facing the flow path 35 in a portion where no hole is formed), or may be the height at a representative position. The representative position can be an arbitrarily selected position of the substrate 10. For example, the representative position may be the center of the substrate 10 forming the flow path 35, or may be a position which is 10% to 50% from an edge of the substrate 10 on the line connecting between this end and the center of the substrate 10. Considering ease of measurement, the representative position is preferred to be a position where an imaginary line perpendicularly extending from the center of another imaginary line connecting between the centers of the first and second holes 21 and 22 in plan view of the cover member 20 intersects the peripheral member 34.

If the microfluidic device 1 includes a wall layer 32, the height of the flow path 35 may be an average of the heights at a plurality of positions on the surface 32a of the wall layer 32 facing the flow path 35, or may be the height at a representative position. The representative position can be an arbitrarily selected position of the wall layer 32. For example, the representative position may be the center of the wall layer 32. Alternatively, the representative position may be a position which is 10% to 50% from an edge of the surface of the wall layer 32 facing the flow path 35 on the line connecting between this edge and the center of the wall layer 32. Considering ease of measurement, the representative position is preferred to be at a position where an imaginary line perpendicularly extending from the center of another imaginary line connecting between the first and second holes 21 and 22 intersects the peripheral member 34.

The term end herein refers to an end in a direction (longitudinal direction) parallel to the imaginary line connecting between the first and second holes 21 and 22 of the microfluidic device 1. The term end refers to a portion contacting the peripheral member 34 described later.

Furthermore, the surface of the cover member 20 defining and facing the flow path 35 is preferred to be smooth. If the surface of the cover member 20 defining and facing the flow path 35 is smooth, generation of air bubbles can be minimized even more when heating the droplets in the droplet holders 11 (microwells 33). In the present specification, being smooth refers to having fine unevenness, i.e., having microscopically fine asperities, as observed by an optical microscope. In the present specification, being smooth does not refer to the cover member 20 not being curved on the inside of the peripheral member 34. In the present embodiment, the cover member 20 may be curved on the inside of the peripheral member 34. For example, the cover member 20 may be curved so as to be projected toward the substrate 10 in the vicinity of the center of the main surface of the cover member 20. In other words, the portion of the flow path 35 contacting the peripheral member 34 may be at a level higher than the flow path 35 in the vicinity of the center of the main surface of the cover member 20. The center of the main surface of the cover member 20 refers to a geometric center of the main surface of the cover member 20.

In the present specification, the term microwell refers to a well with a volume of 10 nanoliter (nL) or less. By allowing each droplet holder 11 to have a volume of a microwell, reactions, such as a digital PCR or digital ICA reaction, can be suitably caused in the microscopic space. Using the method described above, gene mutation or the like, for example, can be detected.

The volume of each droplet holder 11 (microwell 33) is not particularly limited, but is preferred to be 10 femtoliter (fL) or more and 100 picoliter (pL) or less, more preferred to be 10 fL or more and 5pL or less, and most preferred to be 10 fL or more and 2 pL or less. The volume in this range is suitable for holding one to several biomolecules or carriers in one droplet holder 1 (microwell 33) when analyzing a sample described later.

The shape of the microwell 33 is not particularly limited as long as the volume is in the above range. Accordingly, for example, the microwell 33 may have a polygonal shape defined by a plurality of faces (e.g., rectangular parallelepiped, or six- or eight-sided prism), an inverse conical shape, an inverse pyramidal shape (inverse three-, four- five- or six-sided pyramidal shape, or inverse seven or more-sided polygonal pyramidal shape), or the like.

Furthermore, the plurality of microwells 33 may have shapes obtained by combining two or more shapes mentioned above. For example, a part of the plurality of microwells 33 may have a cylindrical shape, and the remaining part may have a reverse conical shape. When the microwells 33 have a reverse conical or reverse pyramidal shape, the bottom surfaces of the cones or pyramids serve as openings allowing communication between the flow path 35 and the microwells 33. In this case, the peak of the reverse conical or reverse pyramidal shape may be truncated so that the microwells 33 have flat bottoms. As another example, bottoms of the microwells 33 may be curves that are protruded toward the openings or may be curves that are recessed.

The ratio of the total area of the openings of the droplet holders 11 (microwells 33) per unit area (usually 1 mm²) of the region in the substrate 10 where the droplet holders are formed is preferred to be 23% or more and 90% or less, more preferred to be 25% or more and 90% or less, even more preferred to be 30% or more and 90% or less, still more preferred to be 35% or more and 80% or less, still more preferred to be 39% or more and 76% or less, and most preferred to be 39% or more and 64% or less. If the ratio of the total area of the openings of the droplet holders 11 (microwells 33) per unit area of the region in the substrate 10 where the droplet holders are formed is in the above preferred range, generation of air bubbles in the flow path can be effectively minimized. This is considered to be because, when heating the aqueous solution in the droplet holders 11 (microwells 33), the pressure applied to the sealant in the flow path 35 can be reduced to some extent due to the total area of the openings not being excessively large.

Hereinafter, the ratio of the total area of the openings of the droplet holders 11 (microwells 33) per unit area of the region in the substrate 10 where the droplet holders are formed may also be termed "opening area ratio".

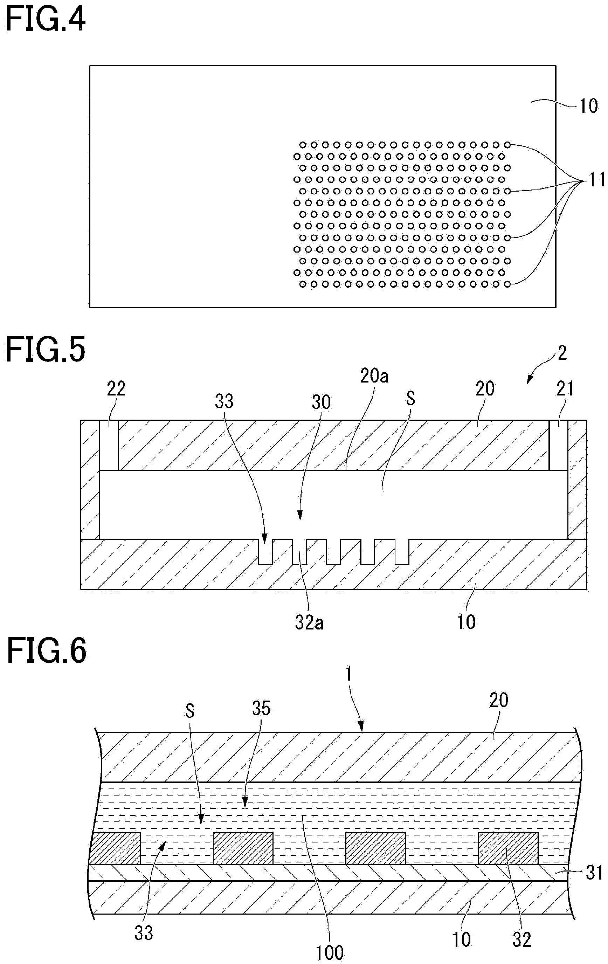

If the region where the droplet holders (microwells 33) are formed extends across the surface of the substrate 10, the total area of the openings of the droplet holders 11 (microwells 33) in the substrate 10 may be approximated to be the same as the area of the region in the substrate 10 defined by the peripheral member. As shown in Fig. 4, the region where the droplet holders 11 (microwells 33) are formed may be present only in a part of the substrate 10. The region where the droplet holders 11 (microwells 33) are formed refers to a region where the droplet holders 11 (microwells 33) are arranged at regular intervals, as shown in Fig. 4, as viewed perpendicularly to the substrate 10. In this case, the total area of the openings of the droplet holders 11 (microwells 33) in the substrate 10 may be approximated to the area enclosed by an imaginary line passing through the centers of the plurality of droplet holders (microwells 33) located at the outermost positions in the region where the droplet holders 11 (microwells 33) are formed. The expression "at regular intervals" does not necessarily mean that the droplet holders are precisely arranged at regular intervals, but may mean, as a matter of course, that they are arranged with manufacturing errors tolerated.

As the area of the region where the droplet holders 11 are formed in the substrate 10 increases with respect to the area of the region therein defined by the peripheral member, the aqueous solution tends to evaporate and air bubbles are easily generated. However, if the height h of the flow path 35 is less than 50 µm as in the present embodiment, generation of air bubbles can be effectively reduced or prevented in the flow path 35.

If a wall layer 32 is present, the depth of the droplet holders 11 (microwells 33) is defined by the thickness of the wall layer 32.

To encapsulate the biomolecule-containing aqueous solution (sample) when the microwells have a cylindrical shape, the thickness of the wall layer 32 is preferred, for example, to be 10 nm or more and 100 µm or less, more preferred to be 100 nm or more and 50 µm or less, even more preferred to be 1 µm or more and 30 µm or less, still more preferred to be 2 µm or more and 15 µm or less, and most preferred to be 3 µm or more and 10 µm or less.

Considering the amount of the aqueous solution to be held, or considering the size or the like of the carriers, such as beads, to which the biomolecules are attached, the dimension of each microwell 33 may be appropriately determined so that one or more biomolecules are held in one microwell.

The ratio of the depth of the droplet holders 11 (microwells 33) to the height h of the flow path 35 is preferred to be 3% or more and 150% or less, more preferred to be 10% or more and 100% or less, even more preferred to be 12% or more and 75% or less, still more preferred to be 15% or more and 75% or less, still more preferred to be 33% or more and 75% or less, and most preferred to be 33% or more and 50% or less. If the ratio is in this range, reducing or preventing generation of air bubbles can be balanced with ease of introduction of an object to be detected into the droplet holders 11 (microwells 33).

The number or the density of the microwells 33 to be provided to the microwell array 30 can be appropriately determined, but it is preferred that the number or the density of the microwells 33 is determined so that the total volume of the microwells 33 will, for example, be 0.2 µL or more and 2.0 µL or less, and more preferably 0.5 µL or more and 1.5 µL or less.

Furthermore, the total volume of the microwells 33 with respect to the volume of the flow path 35 is preferred to be 5% or more and 40% or less, more preferred to be 8% or more and 30% or less, and even more preferred to be 10% or more and 20% or less. If the ratio of the total volume of the microwells 33 to the volume of the flow path 35 is in the preferred range, generation of air bubbles can be further minimized when heating droplets in the microwells 33.

For example, the number of the microwells 33 per 1 cm² may be 10,000 or more and 10,000,000 or less, more preferably 100,000 or more and 5,000,000 or less, and even more preferably 100,000 or more and 1,000,000 or less. In the present specification, the number of the microwells 33 per 1 cm² may be termed the density of the microwells. If the density of the microwells is in this range, the operations of encapsulating the aqueous solution as a sample in a predetermined number of wells may be facilitated. Also, if the density of the microwells is in this range, observation of the wells for analysis after experiments can also be facilitated. For example, for mutations of cell-free DNA, if the ratio of the mutant DNA, as an object to be detected, to wild-type DNA is around 0.01%, it is preferred, for example, to use 1,000,000 to 2,000,000 microwells.

Fig. 1 shows an example of a one-dimensional array in which a plurality of microwells 33 are arrayed in a row. It should be noted that, if numerous microwells are provided as mentioned above, a two-dimensional array may be used in which the plurality of microwells are two-dimensionally arrayed.

The peripheral member 34, which is in a frame shape in plan view, is arranged around the microwell array. The dimension of the peripheral member 34 in the thickness direction of the microfluidic device 1 is larger than that of the wall layer 32. The peripheral member 34, which supports the cover member 20, defines a gap between the cover member 20 and the microwell array to provide the flow path 35. In other words, the flow path 35 is a space sandwiched between the microwell array 30 and the cover member 20 and enclosed by the peripheral member 34.

Materials or the like of the peripheral member 34 are not particularly limited, but may, for example, be a double-sided adhesive tape in which an acrylic adhesive is laminated on both surfaces of a core film made of silicone rubber or an acrylic foam, or may be other materials.

The peripheral member 34 may be formed integrally with the cover member 20. In this case, the peripheral member 34 serves as a step of the cover member 20 and this step defines a gap between the cover member 20 and the microwell array to provide the flow path 35.

The microfluidic device 1 configured as described above can be produced, for example, by the following procedure.

First, a substrate 10 is prepared and a resin layer serving as a wall layer 32 is formed on the surface of the substrate 10. If a bottom layer 31 is provided, it is formed before forming the resin wall layer. Even when a bottom layer 31 is not provided, an anchor layer or the like may be formed, as necessary, on the surface of the substrate 10 to enhance adhesion between the substrate 10 and the resin wall layer.

The resin wall layer may be made of a material obtained by mixing a resin material with colored components. If the resin material is a resist, content of the colored components with respect to the total mass of the resin material and the colored components may, for example, be 0.5 mass% or more and 60 mass% or less. The content is preferred to be 5 mass% or more and 55 mass% or less, and more preferred to be 20 mass% or more and 50 mass% or less. The content of the colored components with respect to the total mass of the resin material and the colored components can be appropriately determined in consideration of the percentage of the photosensitive components or the like contained in the resist, so that a desired pattern can be formed. If the colored components are pigments, the particle size of the pigments is determined so that the predetermined requirements mentioned above are satisfied for the microwells to be formed. In addition to the pigments, a dispersant may be appropriately added to the resin material.

If the resin wall layer formed is made of a material that is a mixture of a resin material and colored components, the resin wall layer may have a hue based on the colored components contained in the resin wall layer.

Next, through holes 32a are formed in the resin wall layer as formed. As mentioned above, use of photolithography enables easy formation of the through holes 32a with high accuracy. If the resin wall layer is formed by injection molding, the resin wall layer and the through holes can be simultaneously formed in a single process. Otherwise, the through holes 32a can be formed by etching or the like using a pattern mask.

After forming the through holes 32a, the resin wall layer is presented as a wall layer 32, thereby completing a microwell array 30.

After that, a peripheral member 34 is arranged around the microwell array 30, followed by arranging a cover member 20 on the peripheral member 34. By integrally bonding the substrate 10, the peripheral member 34, and the cover member 20 together, a microfluidic device 1 is completed. A flow path 35 is formed between the cover member 20 and the substrate 10, being defined by the peripheral member 34. The method of bonding is not particularly limited but may, for example, be bonding using an adhesive, a double-sided tape, laser welding, heat sealing, or the like. If the sample analysis method using the microfluidic device 1 includes a heating reaction, bonding using an adhesive, a double-sided tape, or laser welding is preferred because the device that has been formed using such bonding can sufficiently endure pressure rise in the inner space S due to heating.

In the microfluidic device 1, the substrate 10 may be formed integrally with the wall layer 32, and the peripheral member 34 may be formed integrally with the cover member 20. Fig. 5 shows a microfluidic device 2 in which a substrate 10 is formed integrally with a wall layer 32, and a peripheral member 34 is formed integrally with a cover member 20. The microfluidic device 2 can be prepared by arranging a substrate 10 that is formed integrally with the wall layer 32, at a cover member 20 that is formed integrally with a peripheral member 34, and bonding a step formed by forming the peripheral member 34 integrally with the cover member 20 to the substrate 10 that is formed integrally with the wall layer 32. The step formed on the cover member 20 defines a flow path 35 between the cover member 20 and the substrate 10.

The configuration of the microfluidic device 2 other than the configuration in which the substrate 10 is formed integrally with the wall layer 32 and the peripheral member 34 is formed integrally with the cover member 20, is the same as the configuration of the microfluidic device 1 described above.

As another mode of the microfluidic device, a substrate 10 and a wall layer 32 may be provided as separate components, and a peripheral member 34 may be formed integrally with a cover member 20. In this case as well, the configuration of the microfluidic device other than the configuration in which the peripheral member 34 is formed integrally with the cover member 20, is the same as the configuration of the microfluidic device 1 described above.

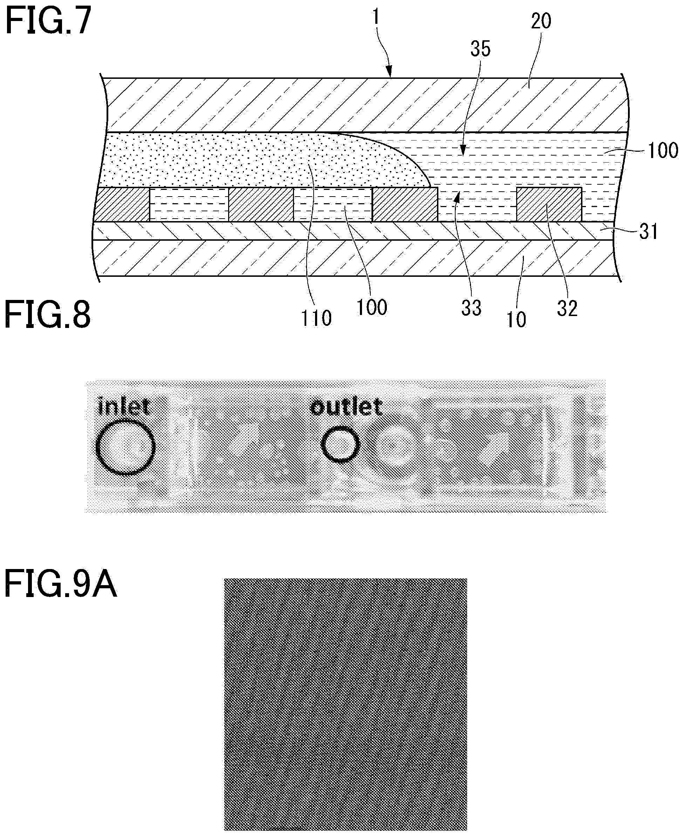

Next, referring to Figs. 6 and 7, a sample analysis method of the present embodiment using the microfluidic device 1 according to the present embodiment will be described.

The sample analysis method of the present embodiment is a method using the microfluidic device 1 according to the present embodiment, including:
introducing a sample-containing aqueous solution into the flow path 35 and allowing the droplet holders 11 to accommodate the aqueous solution;
introducing a sealant into the flow path 35 for replacement with the aqueous solution present in the flow path 35 and encapsulating the aqueous solution in the droplet holders 11;
causing a reaction in the droplet holders 11 and generating a signal for detection; and
detecting the signal.

The aqueous solution may contain water, a buffer solution, a detection reaction reagent, and the like. Furthermore, the aqueous solution may contain an enzyme. For example, if the sample is a nucleic acid, a PCR method, ICA method, LAMP method (trademark, loop-meditated isothermal amplification), TaqMan method (trademark), fluorescent probe method, or the like may be used. For example, if the sample is a protein, an ELISA method (trademark) or the like may be used. Furthermore, the aqueous solution may contain additives, such as a surfactant.

Examples of the buffer solution may include a Tris-HCI buffer, acetic acid buffer, and phosphate buffer.

Examples of the enzyme may include DNA polymerase, RNA polymerase, reverse transcriptase, and flap endonuclease.

Examples of the surfactant may include Tween 20 (which may also be termed polyoxyethylene sorbitan monolaurate), Triton-XlOO (which may also be termed polyethylene glycol mono-4-octylphenyl ether (n = approximately 10)), glycerol, octylphenol ethoxylate, and alkyl glycoside.

The microfluidic device of the present embodiment can suitably accommodate the aqueous solution in the wells in the case where temperature of the encapsulated aqueous solution is changed when detecting, for example, genetic mutations or the like. The range of temperature, i.e., the range of temperature change from lower limit to upper limit may, for example, be 0°C to 100°C, preferably 20°C to 100°C, more preferably 20°C to 90°C, even more preferably 20°C to 80°C, and most preferably 20°C to 70°C. If the temperature of the aqueous solution encapsulated in the wells is in this range, reactions, such as a PCR reaction or ICA reaction, can be suitably caused in the microscopic spaces.

The microfluidic device of the present embodiment, in which the flow path has a height h of less than 50 µm, can minimize generation of air bubbles when the droplet holders 11 (microwells 33) are heated in the above temperature range.

The sample to be analyzed using the microfluidic device 1 of the present embodiment may, for example, be a sample collected from a living organism, such as blood. The object to be detected using sample analysis may be a PCR product that uses DNA contained in the sample as a template, or may be an artificially synthesized compound (e.g., artificially synthesized nucleic acid imitating DNA as a sample), or other objects. For example, if DNA as biomolecules is an object to be detected, the wells may each have a shape and size suitable for one molecule of DNA.

The sample may be biomolecules such as of DNA, RNA, miRNA, mRNA, protein, lipid, or the like. The lipid may include a lipid bilayer membrane structure. Also, the biomolecules may include cells collected from humans for non-therapeutic purposes, cells collected from animals, microorganisms, or bacteria. If the object to be detected is a biomolecule, the sample analysis method according to an aspect of the present invention can be a biomolecule detection method, and the microfluidic device according to an aspect of the present invention used for the biomolecule detection method can be a biomolecule detection device.

Details of the sample analysis method will be described below. As a preparatory step, a sample-containing aqueous solution to be encapsulated in the microwells is prepared. The sample-containing aqueous solution is a solution that contains water as a main solvent in which an object to be detected is contained. For example, the sample-containing aqueous solution may be a PCR reaction solution that contains SYBR Green as a detection reagent and uses a biological sample as a template, an ICA reaction solution that contains an allele probe, ICA oligo, FEN-1, a fluorescent substrate, or the like, or other solutions. When preparing the aqueous solution, a surfactant may be added so that the sample can easily enter the microwells. Alternatively, beads that specifically recognize an object to be detected may be added so that the object to be detected can be trapped. The object to be detected may float in the aqueous solution without being directly or indirectly bound to carriers, such as beads.

Next, using a syringe or the like, a prepared sample-containing aqueous solution 100 is introduced into the flow path 35 via the first hole 21 so that the sample-containing aqueous solution is held in the droplet holders 11 (this step may also be termed sample supply step). As shown in Fig. 6, the supplied sample-containing aqueous solution 100 is filled in the microwells 33 and the flow path 35. Gas in the flow path 35 is discharged in advance by performing a discharge operation prior to the sample supply step. The discharge operation may be performed by filling a buffer in the flow path 35. The buffer may, for example, be water, buffer-containing water, surfactant-containing water, buffer solution and surfactant-containing water, or the like.

Next, the sample-containing aqueous solution 100 is encapsulated in the droplet holders 11 (microwells 33), as an encapsulation step. Prior to the encapsulation step, a label, such as a fluorescent label, may be attached to the object to be detected in the sample that is contained in the aqueous solution. The fluorescence labeling treatment may be performed prior to the sample supply step, e.g., when preparing the sample, or may be performed after the sample supply step by introducing a fluorescent label into the flow path 35.

In the encapsulation step, a sealant 110 is supplied into the flow path 35 via the first hole 21 using a syringe or the like. The supplied sealant 110 flows in the flow path 35 and, as shown in Fig. 7, pushes the sample-containing aqueous solution 100 present in the flow path 35 toward the second hole 22. Then, the sealant 110 replaces the aqueous medium 100 filled in the flow path 35 so that the flow path 35 is filled with the sealant 110. Consequently, the sample-containing aqueous solution 100 is independently placed only in the individual microwells 33 to thereby complete encapsulation of the sample.

In the present specification, the sealant 110 refers to a liquid used for isolating the introduced aqueous solution between microwells 33 of the microwell array 30 so that the aqueous solution introduced into each microwell will not be mixed with the aqueous solution introduced into other microwells. The sealant may be an oil, for example. Examples of the oil may include FC40 (trademark) manufactured by Sigma Corporation, HFE-7500 (trademark) manufactured by 3M Co., Ltd., and mineral oils used for PCR reactions.

The sealant 110 is preferred to have a contact angle to the material of the wall layer 32 in the range of 5 degrees or more and 30 degrees or less. If the contact angle of the sealant 110 is in this range, the sample can be suitably encapsulated in the microwells 33. The contact angle of the sealant 110 may be measured according to the sessile drop method prescribed in JIS R 3257-1999, for example, using the sealant 110 instead of water. The contact angle may be measured according to the method prescribed in ASTM D5725-1997 instead of the sessile drop method prescribed in JIS R 3257-1999.

Subsequently, a reaction step is performed in which a reaction is caused in the droplet holders 11 (microwells 33) of the microfluidic device 1 to generate signals for detecting the object.

Examples of the signals for detection may include fluorescence, chemiluminescence, color development, potential change, pH change, and the like, but fluorescence is preferred among them.

Prior to the reaction step, the microfluidic device 1 may be placed in a thermal cycler to cause an enzymic reaction, as necessary, such as a PCR reaction or ICA reaction, or other reactions.

For example, the reaction may be a biochemical reaction, or more specifically, an enzymatic reaction. Alternatively, the reaction may be a reaction caused by heating the microfluidic device 1. The heating temperature is appropriately determined according to the reaction, but may, for example, be 60°C or more and 100°C or less. The heating temperature is preferred to be 60°C or more and 90°C or less, more preferred to be 60°C or more and 80°C or less, and even more preferred to be 60°C or more and 70°C or less. The expression "heating temperature" refers to a heating temperature set for a thermal cycler, incubator, or the like for heating the microfluidic device, and does not refer to the actual temperature of the reagent solution in the droplet holders 11 (microwells 33). The expression that "the heating temperature may, for example, be 60°C or more and 100°C or less" refers to that the highest temperature reached may be in the range of 60°C or more and 100°C or less, and the temperature does not have to be constantly 60°C or more and 100°C or less. In other words, the temperature of the microfluidic device 1 may change within the range of temperature mentioned above. An example of the reaction may be a signal amplification reaction. The signal amplification reaction is an isothermal reaction in which the microfluidic device 1 is maintained in a state where a reagent solution containing a signal amplification enzyme is held in the droplet holders 11 (microwells 33) for a predetermined period of time, e.g., at least for 10 minutes, and more preferably for about 15 minutes, under constant temperature conditions, e.g., 60°C or more and 100°C or less, to obtain desired enzymic activity.

Next, signals generated from the droplet holders (microwells 33) with the reaction are detected (also termed detection step). For example, if the signals are fluorescence, the microfluidic device 1 is set on a fluorescence microscope and irradiated with excitation light (electromagnetic waves). The wavelength of the excitation light is appropriately determined according to the fluorescent label used.

Irradiation of electromagnetic waves may be performed from the substrate 10 side of the microfluidic device 1, or may be performed from the cover member 20 side thereof, i.e., from above the microwells 33, or may be performed from any direction. Further, fluorescence or phosphorescence generated as a result of electromagnetic wave irradiation may be detected from the substrate side of the microwell array, or from the wells side, or otherwise, from any direction. For example, if a fluorescence microscope is used, fluorescence or phosphorescence can be easily detected from the substrate 10 side of the microfluidic device 1.

Next, of the plurality of microwells 33 configuring the microwell array 30, the number of the microwells 33 emitting fluorescence or phosphorescence is counted. A fluorescence image of the microwell array 30 may be captured and used for the counting.

For example, a PCR reaction may be caused in the microwell array 30 and SYBR Green fluorescence may be detected in the microwells 33 where PCR amplification has been observed to calculate the ratio of the number of microwells 33 where amplification has been observed to the total number of microwells 33. For example, if the object to be detected is a single nucleotide polymorphism (SNP), frequency of occurrence or the like of SNP can be analyzed by counting the number of the microwells 33 emitting fluorescence.

Another aspect of the present invention encompasses the following modes.
[31] A microfluidic device including a substrate, a cover member located above the substrate, a peripheral member connecting between the substrate and the cover member, a flow path located between the substrate and the cover member and defined by the peripheral member, and one or more droplet holders located on the substrate and establishing connection with the flow path, wherein the flow path has a height of more than 0 µm and 30 µm or less.
[32] The microfluidic device according to [31], wherein the droplet holders are provided on the surface of the substrate.
[33] The microfluidic device according to [31], wherein the device includes at least one first hole for introducing a solution into the flow path, and at least one second hole for discharging the solution from the flow path, and the first hole and the second hole are located sandwiching one or more droplet holders therebetween.
[34] The microfluidic device according to [31] to [33], wherein the droplet holders have bottoms facing the substrate, while the droplet holders have openings facing the cover member, and the flow path is located above the openings.
[35] The microfluidic device according to any one of [31] to [34], wherein each of the droplet holders has a volume of 10 fL or more and 100 pL or less.
[36] The microfluidic device according to any one of [31] to [35], wherein the droplet holders have a total volume of 0.2 µL or more and 2.0 µL or less.
[37] The microfluidic device according to any one of [31] to [36], wherein the ratio of the total volume of the droplet holders to the volume of the flow path is 5% or more and 40% or less.
[38] The microfluidic device according to any one of [31] to [37], wherein the ratio of the depth of the droplet holders to the height of the flow path is 3% or more and 150% or less.
[39] The microfluidic device according to any one of [31] to [38], wherein the ratio of the total area of the openings of the droplet holders per unit area of the region where the droplet holders are formed in the substrate is 23% or more and 90% or less.
[40] The microfluidic device according to any one of [31] to [39], wherein the peripheral member is a step that is formed integrally with the cover member.
[41] A sample analysis method that uses the microfluidic device according to any one of [31] to [40], including introducing a sample-containing aqueous solution into the flow path and allowing the droplet holders to hold the aqueous solution, introducing a sealant into the flow path for replacement with the aqueous solution present in the flow path and encapsulating the aqueous solution in the droplet holders, causing a reaction in the droplet holders and generating a signal for detection, and detecting the signal.
[42] The sample analysis method according to [41], wherein the sample is a biomolecule.
[43] The sample analysis method according to [41] or [42], wherein generating a signal for the detection includes causing the reaction by heating the microfluidic device, and the temperature when heating the microfluidic device is 60°C or more.
[44] The sample analysis method according to any one of [41] to [43], wherein the signal is detected by capturing an image of the microfluidic device.
[45] The sample analysis method according to any one of [41] to [44], wherein the signal is fluorescence.
[46] The sample analysis method according to any one of [41] to [45], wherein the reaction is an isothermal reaction.

### [Examples]

The present invention will be described in more detail by way of examples which in no way are meant to limit the present invention to the following examples.

### (Example 1)

Two resin members were prepared by injection molding, one being a substrate made of COP (ZEONOR 1010R manufactured by Zeon Corporation) and the other being a cover member made of COP (ZEONOR 1010R manufactured by Zeon Corporation). By changing the number of micropores formed in the COP substrate, the total volume of the micropores (i.e., the microwells) was controlled. The cover member was formed integrally with a step and the height of the step was adjusted to 30 µm to provide a flow path having a height of 30 µm.

The micropores each having a diameter of 10 µm and a depth of 15 µm were arranged in a 9,000 mm × 30,000 mm region within the surface of the flow path.

The opening area ratio was taken to be the ratio of the total area of the openings of the micropores per 6.5 mm × 9.0 mm region where the micropores were formed.

The substrate used was formed by injection molding with a thickness of 0.6 mm, with micropores arranged across the surface of the substrate. The height of the flow path was measured using a contact measuring device (Talysurf PGI 1240 manufactured by Taylor Hobson).

The substrate and the step of the cover member were bonded to each other by laser welding to prepare a microfluidic device. A fluorescent reagent (Fluorescein manufactured by Tokyo Chemical Industry Co., Ltd.) having the composition shown in Table 1 was injected into the flow path formed between the substrate and the cover member. Furthermore, the plurality of microwells were individually sealed using a fluorocarbon oil (FC40 manufactured by Sigma Corporation). Although a fluorescence reaction was not carried out in this example, an enzyme was added to the fluorescent reagent in order to create the same conditions as when a fluorescence reaction is carried out.

**[Table 1]**

| Composition of fluorescent reagent | Final concentration |
|---|---|
| Fluorescent reagent | 2 µM |
| MgCl₂ | 20 mM |
| Tris(pH 8.5) | 50 mM |
| Flap endonuclease 1 | 0.1 mg/mL |
| Tween 20 | 0.05% |

The microfluidic device was heated at 66°C for 30 minutes. After leaving the microfluidic device at room temperature, the presence or absence of air bubbles was confirmed from above, i.e., from the cover member side, and an image of the state was captured using a digital camera (CX-4 manufactured by Ricoh Company, Ltd.). Furthermore, a fluorescence image of the micropores was observed through a fluorescence microscope (BZ-710 manufactured by Keyence Corporation) using a 4x objective lens. The exposure time was 20 msec in bright field and 3,000 msec using a fluorescent filter of GFP (green fluorescent protein).

Table 2 shows the rate of generation of air bubbles in the microfluidic device. The rate of generation of air bubbles was calculated by preparing a plurality of microfluidic devices with the same design and dividing the number of microfluidic devices in which air bubbles were present, by the total number of microfluidic devices analyzed. If air bubbles with a size visible to the naked eye were present in a microfluidic device, it was determined that there were air bubbles, and if such bubbles were not present under the same conditions, it was determined that there were no air bubbles. The maximum size of air bubbles visible under the above conditions was approximately 500 µm or more. Even if there were small air bubbles and if these bubbles were each smaller than the distance between the ends of the most adjacent micropores (i.e., microwells) when the microfluidic device was observed through a microscope using a 4x objective lens, the air bubbles were determined not to be present. The reason why such a determination can be made is that the presence of the air bubbles with the above size or less cannot substantially block detection, and that generation of air bubbles is considered to be reduced or prevented.

Consequently, as shown in Table 2, the microfluidic device prepared in Example 1 had a low rate of generation of air bubbles.

Fig. 8 shows results of observing typical air bubbles in a microfluidic device according to a comparative example (flow path height: 100 µm). In Fig. 8, the arrows indicate typical air bubbles generated. Fig. 8 shows that there were air bubbles of a few mm and also that there were observed a plurality of small bubbles of approximately 500 µm.The microfluidic device of Example 1 with a flow path height of 30 µm could reduce the rate of generation of such air bubbles.

Next, the results of fluorescence observation will be shown. As shown in Fig. 9A, the microfluidic device of Example 1 with a flow path height of 30 µm enabled observation of the micropores (i.e., microwells) without the droplets breaking.

### (Example 2)

A microfluidic device was prepared as in Example 1 except that the height of the flow path was 20 µm, and then the rate of generation of air bubbles was measured. Table 2 shows the results. As shown in Table 2, the microfluidic device prepared in Example 2 had a low rate of generation of air bubbles.

### (Comparative Example 1)

A microfluidic device was prepared as in Example 1 except that the height of the flow path was 100 µm, and then the rate of generation of air bubbles was measured. Table 2 shows the results. As shown in Table 2, the microfluidic device prepared in Comparative Example 1 had a high rate of generation of air bubbles.

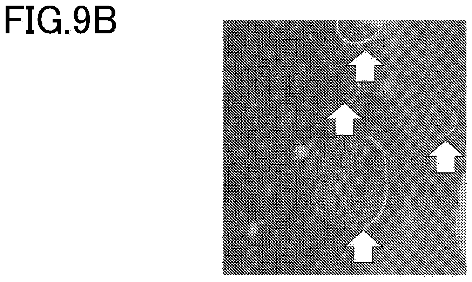

Fig. 9B shows the results of observing fluorescence. The microfluidic device with a flow path height of 100 µm generated many air bubbles and observation of the micropores was difficult. In Fig. 9B, the arrows indicate typical air bubbles generated.

### (Comparative Example 2)

A microfluidic device was prepared as in Example 1 except that the depth of the wells was 3.5 µm and the height of the flow path was 100 µm, and then the rate of generation of air bubbles was measured. Table 2 shows the results. As shown in Table 2, the microfluidic device prepared in Comparative Example 2 had a high rate of generation of air bubbles.

**[Table 2]**

| | Rate of generation of air bubbles (number generated / total number analyzed) | Total volume of wells (µL) | Depth of wells (µm) | Height of flow path (µm) | Volume of flow path (µL) | Opening area ratio (%) |
|---|---|---|---|---|---|---|
| Example 1 | 14 (5/37) | 0.76 | 15 | 30 | 6.57 | 39.5 |
| Example 2 | 14 (3/22) | 0.76 | 15 | 20 | 4.36 | 39.5 |
| Comparative Example 1 | 80 (32/40) | 0.2 | 15 | 100 | 9.22 | 22.7 |
| Comparative Example 2 | 80 (80/100) | 0.04 | 3.5 | 100 | 9.22 | 22.7 |

### (Example 3)

A substrate made of COP (ZEONOR 1010R manufactured by Zeon Corporation) and formed by injection molding was used. The micropores each having a diameter of 5 µm and a depth of 3.5 µm were arranged in a 6.5 mm × 9.0 mm region within the surface of the flow path.

A cover member, which was made of COP (ZEONOR 1010R manufactured by Zeon Corporation) and formed by injection molding, was provided with a PET (polyethylene terephthalate) substrate double-sided tape (No. 5630 BN manufactured by Nitto Denko Corporation) with a thickness of 30 µm as a peripheral member.

A microfluidic device was prepared as in Example 1 except for the above, and then the rate of generation of air bubbles was measured. Table 3 shows the results.

In Example 3, four microfluidic devices were connected to each other to form a set of device group. Three such device groups, i.e., 12 microfluidic devices were prepared.

The "ratio of the region where the wells were formed" was taken to be the ratio of the region where the micropores were formed to the region enclosed by the peripheral member, i.e., the area of the region where the flow path was formed.

### (Comparative Example 3)

A microfluidic device was prepared as in Example 1 except that a PET (polyethylene terephthalate) substrate double-sided tape (product No. 5603 BN manufactured by Nitto Denko Corporation) with a thickness of 50 µm was used as a peripheral member, and then the rate of generation of air bubbles was measured. Table 3 shows the results.

### (Comparative Example 4)

A microfluidic device was prepared as in Example 1 except that a PET (polyethylene terephthalate) substrate double-sided tape (product No. 5603 BN manufactured by Nitto Denko Corporation) with a thickness of 100 µm was used as a peripheral member, and then the rate of generation of air bubbles was measured. Table 3 shows the results.

**[Table 3]**

| | Rate of generation of air bubbles (number generated / total number analyzed) | Total volume of wells (µL) | Diameter of wells (µm) | Depth of wells (µm) | Height of flow path (µm) | Volume of flow path (µL) | Total volume of wells / volume of flow path (%) | Opening area ratio (%) | Ratio of region where wells are formed (%) |
|---|---|---|---|---|---|---|---|---|---|
| Ex. 4 | 0 (0/12) | 0.04 | 5 | 3.5 | 30 | 1.84 | 2.2 | 14.6 | 64.1 |
| Comp. Ex. 3 | 50 (6/12) | 0.04 | 5 | 3.5 | 50 | 4.61 | 0.8 | 14.6 | 64.1 |
| Comp. Ex. 4 | 100 (12/12) | 0.04 | 5 | 3.5 | 100 | 9.22 | 0.4 | 14.6 | 64.1 |

The height of the microfluidic device of Example 4, which corresponded to the thickness of the peripheral member, was 30 µm.The microfluidic device of Example 4 generated no air bubbles.

The microfluidic devices of Comparative Examples 3 and 4 having flow path heights of 50 µm and 100 µm respectively exhibited high rates of generation of air bubbles of 50% and 100%.

As described above, microfluidic devices had a high rate of generation of air bubbles if the flow path height was 50 µm or more, but had a low rate of generation of air bubbles if the flow path height was 30 µm.

### [Industrial Applicability]

According to the present invention, there can be provided a microfluidic device which is capable of reducing or preventing generation of air bubbles when heating microdroplets formed. Furthermore, there can also be provided a sample analysis method with which generation of air bubbles can be reduced or prevented and the efficiency of detecting a sample can be improved when optically detecting the sample by heating microdroplets formed. If biomolecules are used as a sample, the biomolecules are required to be heated at high temperature for a given period of time or more. According to the present invention, generation of air bubbles can be efficiently minimized even in such a case.

### [Reference Signs List]

- 1, 2: Microfluidic device
- 10: Substrate
- 11: Droplet holder
- 20: Cover member
- 30: Microwell array
- 32: Wall layer
- 33: Microwell
- 35: flow path
- 100: Aqueous solution
- 110: Sealant

## Claims

1. A microfluidic device comprising a flow path, and one or more droplet holders connected to the flow path, wherein the flow path has a height of more than 0 µm and 30 µm or less.

2. The microfluidic device according to claim 1 further comprising a flat substrate, wherein the flow path is located above the flat substrate, and the droplet holders are present on the substrate.

3. The microfluidic device according to claim 2 further comprising a cover member, wherein the flow path is a space sandwiched between the cover member and the substrate.

4. The microfluidic device according to claim 2 or 3, wherein a ratio of a total area of openings of the droplet holders per unit area of a region where the droplet holders are formed in the substrate is 23% or more and 90% or less.

5. The microfluidic device according to any one of claims 1 to 4, wherein the droplet holders are a plurality of droplet holders.

6. The microfluidic device according to any one of claims 1 to 5, wherein each of the droplet holders has a volume of 10 fL or more and 100 pL or less.

7. The microfluidic device according to any one of claims 1 to 6, wherein a total volume of the droplet holders is 0.2 µL or more and 2.0 µL or less.

8. The microfluidic device according to any one of claims 1 to 7, wherein a ratio of a total volume of the droplet holders to a volume of the flow path is 5% or more and 40% or less.

9. The microfluidic device according to any one of claims 1 to 7, wherein a ratio of a depth of the droplet holders to a height of the flow path is 3% or more and 150% or less.

10. A sample analysis method that uses the microfluidic device according to any one of claims 1 to 9, comprising:
introducing a sample-containing aqueous solution into the flow path and allowing the droplet holders to accommodate the aqueous solution;
introducing a sealant into the flow path for replacement with the aqueous solution present in the flow path and encapsulating the aqueous solution in the droplet holders;
causing a reaction in the droplet holders and generating a signal for detection; and
detecting the signal.

11. The sample analysis method according to claim 10, wherein the sample is a biomolecule.

12. The sample analysis method according to claim 10 or 11, wherein generating a signal for the detection includes causing the reaction by heating the microfluidic device, and the temperature when heating the microfluidic device is 60°C or more.

13. The sample analysis method according to any one of claims 10 to 12, wherein the signal is detected by capturing an image of the microfluidic device.

14. The sample analysis method according to any one of claims 10 to 13, wherein the signal is fluorescence.

15. The sample analysis method according to any one of claims 10 to 14, wherein the reaction is an isothermal reaction.
